# EUROPEAN PATENT APPLICATION

(11) **EP 4 265 219 A1**
(43) Date of publication of application: **25.10.2023**
(21) Application number: 22193020.9
(22) Date of filing: 30.08.2022
(51) Int. Cl.: A61B 90/00, A61B 50/00, B25J 19/00, B25J 19/06, A61B 34/30

(54) **A CASING ASSEMBLY FOR COVERING A ROBOT ARM IN A SURGICAL ROBOTIC SYSTEM**

(30) Priority: 21.04.2022 EP 22169326
(71) Applicant: Microsure B.V., 5692 EA Son (NL)
(72) Inventor: VAN GEVEN, Lars, 5382 KC Vinkel (NL); VAN LOON, Sebastian, 5653 EX Eindhoven (NL); KURSTEN, Jacobus Marinus Andreas, 5725 CN Asten Heusden (NL); BOSSCHER, Kasper, 2616 GX Eindhoven (NL); CHATROU, Martijn Lambertus Laurentius, 5692 VN Son en Breugel (NL); VAN DER WALLE, Dirk, 6861 BB Oosterbeek (NL); DENASI, Alper, 5623 BB Eindhoven (NL); BEZEMER, Paul, 5702 TC Helmond (NL)
(74) Representative: DTS Patent- und Rechtsanwälte Schnekenbühl und Partner mbB

(57) **Abstract**

Provided is a casing assembly for covering a robot arm in a surgical robotic system. The robot arm has a plurality of operatively connected arm modules, each enclosed in a metallic housing. The casing assembly comprising at least one covering element for covering said plurality of arm modules of the robot arm, wherein the at least one covering element is configured to mount onto, respectively demount from, said housing of the plurality of arm modules; said at least one covering element comprising a base plate and a sidewall extending therefrom, thereby defining an inner surface for covering an area of the housing of the plurality of arm modules, wherein said inner surface is matched to the corresponding area to be covered; said at least one covering element comprising projection elements projecting from the inner surface thereof, wherein the projection elements are configured to provide for a gap between the base plate and the housing of the plurality of arm modules when the at least one covering element is mounted thereon; said at least one covering element being made of a material for facilitating electrical and thermal insulation of the housing of the plurality of arm modules.

## Description

The present invention generally relates to a casing assembly for covering a robot arm in a surgical robotic system. Specifically, the present invention relates to a casing assembly comprising at least one covering element for thermal and electrical insulation of a housing of the robot arm. More specifically, the present invention relates to a use of the casing assembly in a method for reducing the temperature of the robot arm.

With advances in robotic technology, surgical robotic systems offer a unique opportunity to perform delicate surgical and microsurgical procedures in an extremely precise and controlled manner. Such surgical robotic systems inter alia include an articulated robot arm and a robotic gripper (the so-called end effector) attached to one end of the robot arm for holding a variety of surgical instruments.

The robot arm in turn comprises a plurality of arm sections that are pivotally connected to each other via joints. The arm sections and joints, each include electronics and mechanical components, e.g. servomotors, clutches and brakes, drive belts and electronic boards, that are typically disposed within a metallic housing.

Often the heat produced by the electronics and mechanical components during use of the surgical robotic systems causes an increase of the surface temperature of the metallic housing. The temperature of the housing may therefore reach values that may cause burn injuries of different degrees when coming into contact with the intact skin of patients or system operators. In addition, such temperature rises may be more problematic when the robot arm is encased by a sterile drape.

When performing surgical procedures, the robot arm may repeatedly come into contact with the intact skin of the patient. The medical staff or system operators may also come into contact with the robot arm either accidentally or intentionally, for instance, while changing the surgical instruments coupled to the end-effector, or when re-positioning the robot arms manually

Accordingly, the safety regulations set by the medical electrical standard IEC 60601-1 requires that the temperature of the outer housing of the medical devices (such as a robot arm) should be kept below certain temperatures. Further, the medical devices must have protection means to meet the standard limits specified for the medical device. The protection means include a basic or double electrical isolation barrier depending on the types of contact (e.g. accidental or intentional) and the contact duration.

One known approach to generally reduce the temperature of the housing is to equip the robotic arm with a cooling system for cooling the components of the robot arm while in use. In one other known approach, the metallic housing of the robot arm is replaced by a plastic housing to thereby reduce a temperature rise thereof.

Further known approaches to address the safety regulations are based on reducing the generation of heat by lowering the power of the electronics and the mechanical components employed in the surgical robotic systems. Yet one another known approach to ensure the safety of the patients resides in preventing possible contacts of the robot arm with the patient's skin. This is achieved by increasing the length of the robot arm, in particular at a section that is attached to the robotic gripper.

The known approaches, however, all have a number of deficiencies that not only limit the dexterity and functionality of the robot arm, but also compromise the performance and the reliability of the surgical robotic systems.

It would therefore be advantageous to provide a solution that would ensure the safety of the patients and operators without having to limit the performance of the robot arm and the reliability of the surgical robotic systems. Further, it would be advantageous to provide a solution that would allow to thermally (and electrically) isolate specific areas on the housing of the robot arm, while the cooling device (provided in the robot arm) is still in use. It would be even more advantageous to provide for a solution that improves the heat dissipation in the robot arm thereby allowing to employ more powerful electronics and mechanical components, which may result in a better performance of the robot arm.

The present invention therefore aims to achieve the advantageous set forth above by overcoming, or at least reducing, the drawbacks of the known surgical robotic systems.

The present disclosure is directed to aspects and embodiments of a casing assembly for covering a robot arm in a surgical robotic system, and to the use of the casing assembly in a method for reducing the temperature of the robot arm. The robot arm has a plurality of operatively connected robot arm modules, each enclosed in a metallic housing.

According to a first broad aspect of the present invention, the casing assembly comprises at least one covering element for covering the plurality of arm modules of the robot arm, wherein the at least one covering element is configured to independently mount onto, respectively demount from, the housing of the plurality of arm modules. The at least one covering element comprises a base plate and a sidewall extending therefrom, thereby defining an inner surface for covering an area of the housing of the plurality of arm modules, wherein the area of said inner surface is matched to the corresponding area to be covered. The at least one covering element further comprises projection elements projecting from said inner surface thereof, wherein the projection elements are configured to provide for a gap between the base plates and the housing of the plurality of arm modules when said at least one covering element is mounted thereon. Said at least one covering element is made of a material for facilitating electrical and thermal insulation of the housing of the plurality of arm modules. The plastic covering element advantageously creates an air gap between the housing of the plurality of the arm modules while providing for thermal insulation.

The casing assembly of the present invention is configured to increase the safety of the patients and operators. This is achieved by the provision of at least one covering element that is designed and configured to cover predetermined areas on the housing of the plurality of robot arm modules, which areas are likely to get heated from the heat generated by the electronics and the mechanical components employed in the robot arm.

Further, the casing assembly of the present invention is configured to provide for a gap (i.e. an air gap) between said at least one covering element and the housing of the robot arm modules. The air gap serves the purpose of an additional thermal insulation layer for the robot arm. The provision of the air gap and/or the covering element facilitate a double thermal barrier for the housing of the plurality of robot arm modules.

The casing assembly of the present invention in particular provides for an improved approach to address the thermal constraints limiting the dexterity and functionality of the robot arm. The temperature of the housing of the robot arm modules is reduced without having to decrease the power consumption of the components disposed within the housing of the robot arm modules. Moreover, the temperature reduction is achieved without having to modify the housing of the robot arm modules, e.g. for increasing the surface area thereof.

The casing assembly of the present invention is such configured that it neither interferes with the operation of the existing electronics and mechanical components, nor does it hinder operation of the cooling device that may be required.

The at least one covering element of the casing assembly is configured to be independently mounted onto, respectively demounted from, the housing of the respective robot arm modules. In this way, the covering element can easily be removed or replaced. Optionally, the casing assembly may comprise a plurality of the covering elements, each being configured to be independently mounted onto (demounted from) the housing of the robot are modules.

The inner surface of said at least one covering element (i.e. the interior area thereof) is geometrically matched to the corresponding area to be covered on the housing of the robot arm modules. That is, the shape and dimensions of the base plates and the sidewalls are fitted to the ones of the predetermined areas on the housing of the robot arm modules.

In embodiments of the present invention, said at least one covering element is made of material such as plastic, preferably, polylactic acid (PLA), acrylonitrile butadiene styrene (ABS) or nylon.

In embodiments of the present invention, the at least one covering element comprises a plurality of orifices formed on the corresponding base plates for transferring the heat generated within the housing of the plurality of arm modules. The plurality of orifices is configured to function as ventilation holes improving the convection of the heat to the ambient atmosphere, i.e. the operating theatre. Alternatively or additionally, the at least one covering element comprises a plurality of slits (e.g. cut-outs) that is formed on the sidewalls extending from the corresponding base plates.

In embodiments of the present invention, the casing assembly (comprising the at least one covering element) is configured to reduce the temperature of the housing of the plurality of robot arm modules.

In embodiments of the present invention, the casing assembly is configured to reduce the temperature of the housing of the plurality of robot arm modules, i.e. the outermost surface of the housing, by about 10°C to 25°C.

In embodiments of the present invention, the at least one covering element may further comprise openings formed on the base plates and/or the sidewalls for receiving mechanical fasteners. In this way, the at least one covering element is securely mounted on (i.e. fastened to) the housing of the plurality of arm modules.

The mechanical fasteners include any suitable mechanical fasteners such as bolts and nuts, screws, studs, and rivets.

Alternatively or additionally, the at least one covering element is configured to be snap-fitted onto the housing of the plurality of arm modules.

In embodiments of the present invention, the base plates of the at least one covering element comprise an element for disengaging components, e.g. mechanical and/or electrical components, disposed within the housing of the plurality of arm modules.

The elements for disengaging are, for example, in the form of a push knob. Optionally, the push knob is configured to disengage all brakes disposed within the housing of the plurality of arm modules.

In embodiments of the present invention, the at least one covering element comprises a sidewall that is configured to circumscribe the respective base plate thereof.

In embodiments of the present invention, the at least one covering element is configured to envelop the corresponding housing of the plurality of the robot arm modules.

In embodiments of the present invention, the at least one covering element comprises a base plate having a shape similar to the shape of the cross-section of the housing of the plurality of arm modules.

In embodiments of the present invention, either one the base plate or one the sidewall of the at least one covering element has a thickness between 0.5 and 5mm.

In embodiments of the present invention, the sidewall of the at least one covering element has a height between 1 and 5 mm.

In embodiments of the present invention, the gap between the base plate and the corresponding housing of the plurality of arm modules is between 1 and 5 mm, preferably between 1.5 and 2.5 mm.

In embodiments of the present invention, the base plate, sidewall and projection elements of the respective covering element are configured to be formed in one piece through injection moulding.

According to a second broad aspect of the present invention, a casing assembly is provided for use in a method for reducing the temperature of a housing of a plurality of arm modules of a robotic arm in a surgical robotic system.

In embodiments, the method for reducing the temperature of the housing of the plurality of arm modules comprises mounting the casing assembly onto the housing of the plurality of arm modules, and securing the at least one covering element of the casing assembly on the housing of the plurality of arm modules.

The at least one covering element is configured to be fastened through the openings formed on the sidewalls onto said housing to thereby cover predetermined areas on the housing of the plurality of arm modules. Alternatively, the at least one covering element is secured onto the housing of the robot arm using a snap-fit.

Exemplary non-limiting embodiments are described with reference to the accompanying drawings in which:
**Fig. 1** illustrates a schematic view of a robot arm of a surgical robotic system having a casing assembly according to the present invention mounted thereon;
**Fig. 2** illustrates a perspective view of the casing assembly including at least one covering element;
**Fig. 3** illustrates another perspective view of the casing assembly according to the present invention .

Although specific configurations and arrangements are discussed, it should be understood that this is done for illustrative purposes only. A person skilled in the pertinent art will recognize that other configurations and arrangements are also feasible without departing from the scope of the present invention.

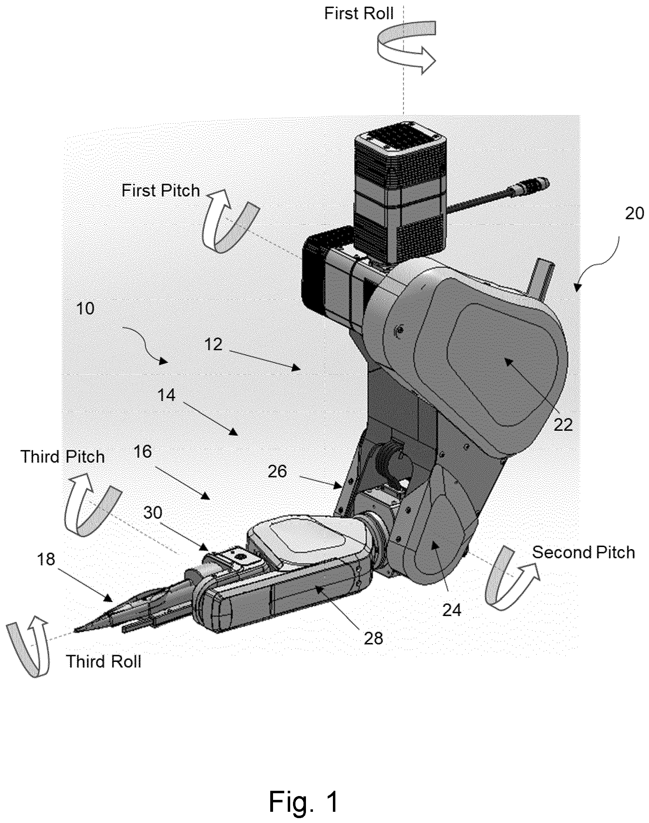

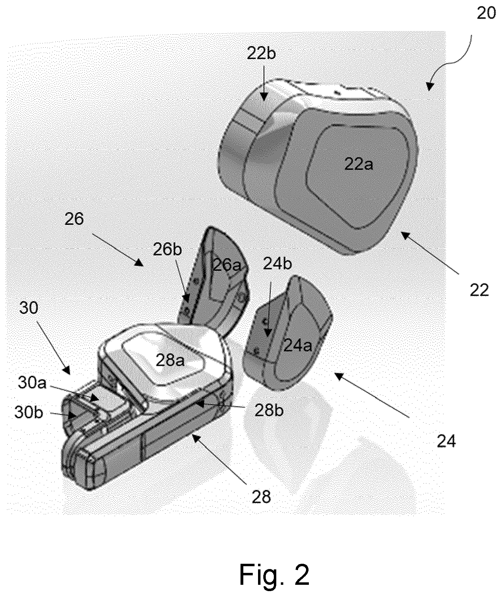

**Fig. 1** illustrates a schematic view of a casing assembly 20 mounted on a robot arm 10 and **Fig. 2** illustrates an exemplary view of the casing assembly 20 in isolation (i.e. when it is not mounted onto the robot arm).

For illustration purposes, the Figures indicate a plurality of the covering elements 22, 24, 26, 28, 30, however the casing assembly of the present invention may only comprise one covering element.

Each covering element is configured to be securely mounted on the housing 12, 14, 16 of the respective arm modules of the robot arm.

As indicated in **Fig. 1****,** the robot arm 10 includes a plurality of arm modules, each enclosed in a housing 12, 14, 16. The housing is typically made of metal, e.g. aluminum, stainless steel.

The plurality of robot arm modules is operatively connected and configured to facilitate the translational and rotational movement of a robotic gripper 18.

Each of the plurality of arm modules is equipped with electronics and mechanical components, such as servo motors, driving belts, encoders and brakes, and printed circuit boards, that are disposed within the corresponding housing of the corresponding plurality of arm modules.

When performing delicate surgical procedures, e.g., microsurgical procedures, it is required that the robot arm performs complex movements to precisely manipulate the surgical instruments (attached to the robotic gripper) within a small incision of around 1 to 2 cm.

Accordingly, during the surgical procedures, the electronics, and mechanical components within the housing of the robot arm modules generate heat. The generated heat causes the temperature of the housing of the plurality of robot arm modules to rise.

The casing assembly 20 for example may include the covering elements 22, 24, 26, 28 for covering areas on the housing 12, 14, 16 of the plurality of arm modules as indicated in **Figs. 1** **and** **2****.**

Although a plurality of covering elements is indicated, it is also anticipated that the casing assembly 20 to include only one covering element.

The covering elements 22, 24, 26, 28, 30, each are configured to be mounted onto, respectively be demounted from, the housing of the corresponding robot arm modules.

The mounting onto, respectfully demounting from, of the covering elements 22-30 can be conducted individually and independent of each other.

Optionally, the covering elements are configured to envelop the housing of the plurality of the robot arm modules. In this way, the surface of the corresponding housing which may have the highest temperature rise is encased by the covering elements.

For example, in the robot arm as shown in **Fig. 1****,** the housing 12 of the robot arm comprises one covering element 22 and the housing 14 comprises two covering elements 24, 26.

The covering element 22 is configured to be mounted onto an area of the housing where two different rotational axes of the robot arm are jointed together, i.e. the so-called first roll and pitch axes as indicated in **Fig. 1****.**

Further, the covering elements 24 and 26 are configured to be mounted onto opposite sides of the housing 14 where the robot arm provides for a third and fourth rotational axes, i.e. a second roll axis (not shown) and a second pitch axis.

The housing 16 includes two cover elements 28 and 30 which are configured to be mounted onto the robot arm where the robot arm, respectively, provides for fifth and sixth rotational axes, i.e. third roll and pitch axes as indicated in **Fig. 1****.**

**Fig. 2** indicates that the covering elements, each comprises a base plate 22a, 24a, 26a, 28a and a sidewall 22b, 24b, 26b,28b.

The sidewalls 22b-28b are configured to extend from the associated base plates 22a-28a.

Optionally, the sidewalls 22b, 28b are configured to circumscribe the corresponding base plates 22a, 28a of the covering elements 22, 28.

The thickness of the base plates and the sidewalls may vary between 0.5 and 3 mm.

To sufficiently cover (thermally isolate) the housing of the plurality of the robot arm modules, the sidewalls 22b-28b are of a predetermined height depending on the geometry of the housing 12, 14, 16 of the plurality of arm modules.

The height of the sidewalls may vary between 1 and 5 mm.

Further, each of the covering elements 22, 24, 26, 28, 30 is configured to provide for an inner surface that is defined by the corresponding base plates and sidewalls.

The shapes of the base plates 22a-28a vary depending on the corresponding shape of the predetermined areas on the housing 12, 14, 16 that must be covered as evident from **Figs. 1** **and** **2****.**

The base plates may be of shapes similar to the shape of the cross-section of the housing of the plurality of arm modules at the areas to be covered.

The geometry (the shape and dimensions) of the base plates 22a-28a and the sidewalls 22b-28b are designed such that the therewith defined inner surfaces (interior surface), each are configured to sufficiently cover the predetermined areas on the housing 12, 14, 16 of the plurality of arm modules.

The areas of the inner surfaces are thus matched to the predetermined areas on the housing.

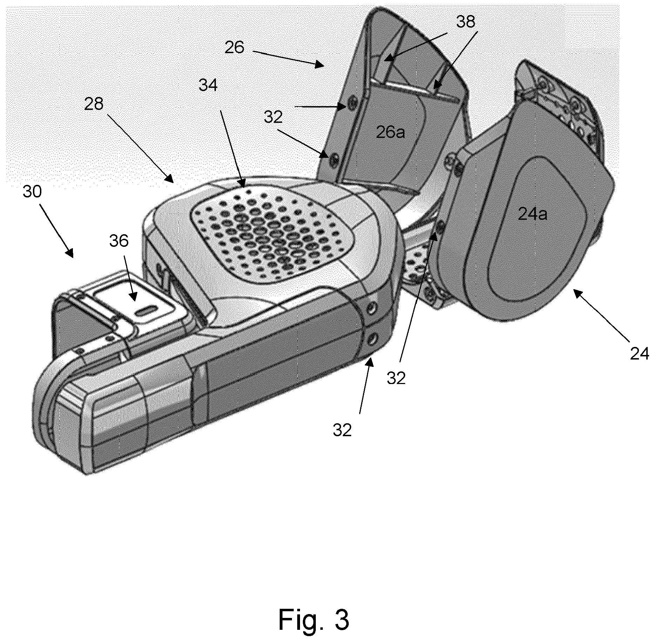

**Fig. 3** illustrates a further view of the covering elements.

The covering elements 22, 24, 26, 28, 30 further comprise projection elements 38 that are projecting from the inner surface of thereof (not all the projection elements are shown).

The projection elements 38 are configured to provide for a gap (i.e. air gap) between the respective base plate and the housing of the plurality of arm modules when the plurality of covering elements are mounted thereon.

Also indicated in **Fig. 3** are openings 32 that are formed on the base plates and/or the sidewalls for receiving mechanical fasteners. The covering elements 22, 24, 26, 2830, each is configured to be securely mounted onto the housing 12, 14, 16 of the plurality of arm modules.

The mechanical fasteners include any suitable mechanical fasteners, such as bolts and nuts, screws, studs, and rivets.

Optionally, each of the covering elements 22, 24, 26, 28, 30 is configured to be mounted onto the housing of the plurality of arm modules by way of snap-fits.

The covering elements 22, 24, 26, 28, 30, each is made of a material that is configured to facilitate electrical and thermal insulation of the housing 12, 14, 16 of the plurality of arm modules.

The plurality of covering elements 22, 24, 26, 28,30, each is made of plastic materials and preferably include polylactic acid (PLA), acrylonitrile butadiene styrene (ABS) or nylon.

The base plate, sidewall, and projection elements of each of the covering elements 22, 24, 26, 28,30 are configured to be formed in one piece through injection moulding.

**Fig. 3** further indicates that at least one covering element 28 may further comprise a plurality of orifices 34 formed on the base plate thereof.

The plurality of the orifices is serving the purpose of direct transfer of the heat generated by the electronics and mechanical components to the ambient.

This allows further reduction of the temperature of the housing 12, 14, 16 of the plurality of arm modules.

Optionally, a plurality of slits or cut-outs is formed on the sidewalls. This will further enhance heat transfer from the housing. 12, 14, 16 of the plurality of robot arm modules

Further, it is envisaged that the covering element 30 includes a disengaging element 36.

The disengaging element 36 is configured to disengage the electronic and mechanical components disposed within the housing 12, 14, 16 of the plurality of arm modules.

The disengaging element 36 is, for example, in the form of a push knob. The push knob is configured to disengage, for example, all brakes disposed within the housing 12, 14, 16 of the plurality of arm modules.

In particular by means of the casing assembly of the present invention, the areas on the housing of the robot arm (the outmost surface of the housing) that may get heated and may potentially touch the intact skin of the patient and/or operators are reliably covered and thermally isolated.

The covering elements are easily accessible and can be removed without disassembling the robot arm.

In addition, the casing assembly 20 is configured to be used in a method for reducing the temperature of the housing of the plurality of arm modules of the robot arm.

In particular, the temperature of the outermost surface of the housing of the plurality of robot arm modules, which are accessible by the system operators, can be reduced by about 10°C to 25°C.

### LIST OF REFERENCE SIGNS

- 10: robot arm
- 12, 14, 16: housing of the robot arm modules
- 18: robotic gripper
- 20: casing assembly
- 22, 24, 26, 28, 30: covering elements
- 22a, 24a, 26a, 28a, 30a: base plate of the covering elements
- 22b, 24b, 26b, 28b, 30b: sidewall of the covering elements
- 32: openings
- 34: a plurality of orifices
- 36: disengaging elements
- 38: projection elements

## Claims

1. A casing assembly (20) for covering a robot arm (10) in a surgical robotic system, the robot arm (10) having a plurality of operatively connected arm modules, each enclosed in a metallic housing (12, 14, 16), the casing assembly (20) comprising:
at least one covering element (22, 24, 26, 28, 30) for covering said plurality of arm modules of the robot arm, wherein the at least one covering element (22, 24, 26, 28, 30) is configured to mount onto, respectively demount from, said housing (12, 14, 16) of the plurality of arm modules;
said at least one covering element (22, 24, 26, 28, 30) comprising a base plate (22a, 24a, 26a, 28a, 30a) and a sidewall (22b, 24b, 26b, 28b, 30b) extending therefrom, thereby defining an inner surface for covering an area of the housing (12, 14, 16) of the plurality of arm modules, wherein said inner surface is matched to the corresponding area to be covered;
said at least one covering element (22, 24, 26, 28, 30) comprising projection elements (38) projecting from the inner surface thereof, wherein the projection elements (38) are configured to provide for a gap between the base plate and the housing (12, 14, 16) of the plurality of arm modules when the at least one covering element is mounted thereon;
said at least one covering element (22, 24, 26, 28, 30) being made of a material for facilitating electrical and thermal insulation of the housing of the plurality of arm modules.

2. The casing assembly (20) according to claim 1,
**characterized in that**
said material of said at least one covering element is made of plastic, preferably polylactic acid (PLA), acrylonitrile butadiene styrene (ABS) or nylon.

3. The casing assembly (20) according to claim 1 or 2,
**characterized in that**
said at least one covering element (22, 24, 26, 28, 30) comprises a plurality of orifices (34) formed on the base plate (22a, 24a, 26a, 28a, 30a) for transferring the heat generated within the housing (12, 14, 16) of the plurality of arm modules, optionally said at least one covering element comprises a plurality of slits formed on the sidewalls thereof.

4. The casing assembly (20) according to one of the preceding claims, **characterized in that**
said at least one covering element (22, 24, 26, 28, 30) is configured to reduce the temperature of the housing (12, 14, 16) of the plurality of robot arm modules by 10°C to 25°C.

5. The casing assembly (20) according to one of the preceding claims, **characterized in that**
said at least one covering element (22, 24, 26, 28, 30) further comprises openings (32) formed on the base plate (22a, 24a, 26a,28a, 30a) and/or the sidewall (22b, 24b, 26b, 28b, 30b) for receiving mechanical fasteners.

6. The casing assembly (20) according to one of the preceding claims, **characterized in that**
said at least one covering element (30) comprises an element (36) for disengaging components disposed within the housing of the plurality of arm modules.

7. The casing assembly (20) according to claim 6,
**characterized in that**
said element (36) for disengaging includes a push knob, preferably configured to disengage all brakes disposed within the housing (12, 14, 16) of the plurality of arm modules.

8. The casing assembly (20) according to one of the preceding claims, **characterized in that**
said at least one covering element (22, 28) comprises a sidewall (22b, 28b) configured to circumscribe the base plate (22a, 22b) thereof.

9. The casing assembly (20) according to one of the preceding claims, **characterized in that**
said at least one of the covering element (30) is configured to envelop the corresponding housing of the plurality of the robot arm modules.

10. The casing assembly (20) according to one of the preceding claims, **characterized in that**
said at least one covering element (22, 24, 26, 28, 30) comprises a base plate (22a, 24a, 26a,28a, 30a) having a shape similar to the shape of the cross-section of the housing (12, 14, 16) of the plurality of arm modules.

11. The casing assembly (20) according to one of the preceding claims, **characterized in that**
the base plate (22a, 24a, 26a,28a, 30a) and the sidewall (22b, 24b, 26b, 28b, 30b) of said at least one covering element (22, 24, 26, 28, 30) have a thickness between 0.5 and 5 mm.

12. The casing assembly (20) according to one of the preceding claims, **characterized in that**
the sidewall (22b, 24b, 26b, 28b, 30b) of said at least one covering element (22, 24, 26, 28, 30) has a height between 1 and 5 mm.

13. The casing assembly (20) according to one of the preceding claims, **characterized in that**
said gap between the base plate (22a, 24a, 26a, 28a, 30a) and the corresponding housing (12, 14, 16) is between 1 and 5 mm, preferably between 1.5 and 2.5 mm.

14. The casing assembly (20) according to one of the preceding claims, **characterized in that**
the base plate (22a, 24a, 26a, 28a), sidewall (22b, 24b, 26b, 28b) and projection elements (38) of the at least one covering elements are configured to be formed in one piece through injection moulding.

15. A use of a casing assembly (20) of any of preceding claims in a method for reducing the temperature of a housing (12, 14, 16) of a plurality of arm modules of a robotic arm in a surgical robotic system, the method comprising:
mounting the casing assembly (20) onto the housing (12, 14, 16) of the plurality of arm modules,
securing the at least one covering element (22, 24, 26, 28, 30) of the casing assembly (20) to said housing to thereby cover predetermined areas on the housing (12, 14, 16).
